# EUROPEAN PATENT SPECIFICATION

(11) **EP 0 454 782 B1**
(45) Date of publication and mention of the grant of the patent: **16.04.1997**
(21) Application number: 90902901.9
(22) Date of filing: 05.01.1990
(51) Int. Cl.: G01N 33/574, G01N 33/68, C07K 14/82, C07K 14/435, G01N 33/577

(54) **CANCER RELATED HAPTOGLOBIN (HPR)**
KARZINOMVERWANDTES HAPTOGLOBIN (HPR)
HAPTOGLOBINE APPARENTEE AU CANCER (HPR)

(30) Priority: 17.01.1989 US 297722
(43) Date of publication of application: 06.11.1991
(73) Proprietor: THE JOHNS HOPKINS UNIVERSITY, Baltimore, MD 21205 (US)
(72) Inventor: KUHAJDA, Francis, P., Baltimore, MD 21212 (US); PASTERNACK, Gary, R., Baltimore, MD 21210 (US)
(74) Representative: Dean, John Paul
(86) International application number: US9000026
(87) International publication number: WO9008324

(56) References cited:
- WO-A-89/04963
- JOURNAL OF BIOLOGICAL CHEMISTRY, vol. 260, no. 11, June 1985; N. MAEDA, pp. 6698-6709/
- AMERICAN JOURNAL OF PATHOLOGY, vol. 121, 1985; F.P. KUHAJDA et al., pp. 342- 348/
- CANCER RESEARCH, vol. 47, October 1987; S.-K. OH et al., pp. 5120-5126/
- IMMUNOLOGY, vol. 64, 1988 S.-K. OH et al., pp. 73-79/
- BRITISH JOURNAL OF CANCER, vol. 56, 1987; S. THOMPSON et al., pp. 605-610/

## Description

### Technical Field of the Invention

This invention relates to the field of human proteins which are expressed by neoplastic tissues and not by most normal tissues.

### BACKGROUND OF THE INVENTION

Conventional human haptoglobins have been well studied; they were discovered over 40 years ago and their role is thought to be in the plasma transport of hemoglobin. Haptoglobin synthesis occurs mainly in the liver, although in some cases lymphocyte cultures and brain have been reported to synthesize haptoglobins.

All haptoglobins contain two classes of polypeptide chains, beta chains and alpha chains. Beta chains are almost identical in all haptoglobins, whereas the alpha chains have been found in three forms. Two of the alpha chain forms (Hp1^{f} and Hp1^{s}) differ only in a single amino acid residue at position 54. The third form (Hp2) is longer than either of the other two alpha chains, apparently having arisen by an unequal crossing over to partially duplicate the alpha chain.

In 1985, Maeda (J. Biol. Chem. vol 260 pp 6698-6709, which is expressly incorporated herein), described a stretch of DNA located 2.2 kb downstream from the conventional haptoglobin locus. The DNA sequence indicated that the locus contained an intact gene coding for a theoretical protein whose alpha and beta chains are distinct from, but highly homologous to, conventional haptoglobins. Maeda called this locus Hpr for "haptoglobin related". Several investigators failed to detect expression of this gene. (Oh, et al., Cancer Research, vol. 47, pp. 5120-5126, 1987; Maeda, Journal of Biological Chemistry, vol. 260, pp. 6698-6709, 1985; and Bensi et al., The EMBO Journal, vol. 4, pp. 119-126, 1985).

Kuhajda, et al., Amer. J. Pathol., 12:342-348 (1985), reported that when cells from breast cancer patients were tested for reactivity with an antibody preparation directed against pregnancy-associated plasma protein A (PAPP-A) positive reactivity was highly correlated with poor prognosis. Kuhajda, et al., taught that the prognostic antigen is PAPP-A; there was no suggestion that the antibody reacted with either haptoglobin or haptoglobin-related protein.

### SUMMARY OF THE INVENTION

It is an object of the invention to provide a substantially pure preparation of a polypeptide having an amino acid sequence corresponding to the nucleotide sequence of the Hpr gene and having the ability to stimulate the production of antibodies in a mammal which are immunoreactive with epitopes found on the Hpr gene product but not found on haptoglobin 1 or 2.

It is another object of the invention to provide a preparation of antibodies which is immunoreactive with Hpr protein but not with haptoglobin 1 or 2.

It is yet another object of the invention to provide a preparation of Hpr protein which is substantially purified from Hp1 and Hp2.

It is still another object of the invention to provide a method of producing an Hpr preparation from human cells.

It is yet an additional object of the invention to provide a kit and method for breast cancer prognostication.

It is another object of the invention to provide a method of determining the prognosis of a solid tumor-bearing patient.

It is still another object of the invention to provide a method of assaying Hpr protein in a biological fluid.

It is yet another object of the invention to provide a method of detecting proliferation of tumor cells in a patient.

These and other objects of the invention are provided by one or more of the embodiments described below. In one embodiment a substantially pure preparation of polypeptide is provided having an amino acid sequence corresponding to the nucleotide sequence of the hpr gene. When the preparation of the polypeptide is administered to a mammal, it stimulates the production of antibodies which are immunoreactive with epitopes found on the hpr gene product but which are not found on haptoglobin 1 or 2.

In another embodiment of the invention a preparation of antibodies is provided which is immunoreactive with Hpr protein but not with haptoglobin 1 or 2.

In yet another embodiment of the invention a preparation of Hpr protein is provided which is substantially purified from Hp1 and Hp2.

In still another embodiment of the invention a method of producing an Hpr preparation is provided. Human breast carcinoma cells are tested for immunoreactivity with antibodies which are reactive with epitopes present on Hpr but not present on Hp1 or 2. Immunoreactive breast cancer cells are cultured and a cytoplasmic fraction containing Hpr is harvested from the cultured cells. In an additional embodiment, human decidua or placental tissue is collected. A cytoplasmic fraction containing Hpr protein is harvested from the decidua or placental tissue to provide an Hpr preparation.

In yet an additional embodiment a kit for determining the prognosis of breast carcinoma is provided. The kit comprises a preparation of antibodies which is immunoreactive with epitopes present on Hpr protein but not present on haptoglobin 1 or 2 and a means for detecting the antibodies. A method of prognosticating the course of a solid tumor is also provided wherein histological sections are contacted with a preparation of antibodies which is immunoreactive with Hpr protein but not with haptoglobin 1 or 2. Antibody binding to the sections is determined.

In yet another embodiment of the invention a method is provided for assaying Hpr in a biological fluid. Antibodies immunoreactive with an epitope on Hpr but not found on Hp1 and Hp2 are attached to a solid support which is then contacted with a biological fluid containing an unknown quantity of Hpr under conditions where antibody-antigen complexes form and are stable. The solid support is then contacted with a polypeptide which shares an epitope with Hpr but not with Hp1 and Hp2, under conditions where antibody-antigen complexes form and are stable. The polypeptide also bears a detectable moiety so that the amount of polypeptide bound to the solid support can be quantitated by detecting and quantitating the detectable moiety. A control is performed wherein no biological fluid is contacted with the solid support to determine 100% binding of the polypeptide to the support. The amount of binding reduction observed in the presence of biological fluid is correlated to the amount of Hpr in the biological fluid.

In still another embodiment of the invention, a method is provided of detecting proliferation of tumour cells in a patient. Biological fluid is collected from a patient bearing a tumour. The amount of Hpr in the fluid is quantitated. The amount of Hpr found in the fluid is compared to the amount found in a control fluid collected from a patient with no detectable tumour. An elevated amount of Hpr in the tumour-bearing patient's fluid indicating proliferation of the tumour cells. These and other embodiments are disclosed below in the detailed description of the invention.

Despite the fact that, unlike haptoglobin, Hpr synthesis has not been found in the liver, the present invention provides four different sources which do contain Hpr. Human breast carcinoma cells, human decidua, human placenta, and human pregnancy serum have all been found to contain specifically cross-reactive material with Hpr-specific antibodies. Furthermore, Hpr has been found to be an especially useful diagnostic marker for human solid tumours for predicting the propensity for tumour invasion and early metastasis.

Consequently, the present invention is directed to preparations of the antigenic material from human breast carcinoma cells that are specifically cross-reactive with Hpr-specific antibodies, and to methods of producing the antigenic protein by testing breast cancer cells for reactivity with antibodies to Hpr, culturing cells which test positive and recovering the protein. The present invention provides antibodies which specifically bind the antigenic material from human breast carcinoma cells which are immunologically cross-reactive with a peptide having the first 34 amino acids of Hpr, wherein these antibodies are not reactive with hapoglobin 1 or 2.

The invention also provides methods for determining the prognosis of a solid tumour based on determining whether these antibodies specific for the antigenic material from breast cancer cells bind to histological sections of the tumour. The invention further provides a method for detecting proliferation of tumour cells in a patient comprising obtaining a sample from the patient and quantitating in the sample the amount of a protein specifically bound by antibodies to the material from breast cancer cells that is cross-reactive with Hpr but not with haptoglobin 1 or 2.

The present inventors have discovered and disclose herein for the first time that a polypeptide having the following sequence: 
leu tyr ser gly asn asp val thr asp ile ser asp asp arg phe
pro lys pro pro glu ile ala asn gly tyr val glu lys leu phe arg
tyr gln cys,
 corresponding to the first 34 amino acids of the novel protein Hpr, is an immunogen that will elicit an antibody preparation reactive with an antigen whose presence in a tumour is predictive of worsened prognosis.

### BRIEF DESCRIPTION OF THE DRAWINGS

Figure 1 shows the progressive purification of Hpr protein. Polyacrylamide gel electrophoresis and corresponding western blots are shown of sequential chromatographic purification of plasma proteins reactive with anti-PAPP-A.

Figure 2 shows a polyacrylamide gel electrophoretic separation of immunoreactive heavy and light chains by gradient anion exchange chromatography.

Figure 3 depicts sequences of: (1) Hpr proteip predicted from the gene sequence; (2) haptoglobin 1; and (3) haptoglobin 2, each of which aligns with the N-terminal beta chain sequence of the protein isolated from pregnancy serum.

Figure 4 shows a polyacrylamide gel electrophoretic separation of haptoglobin 1, haptoglobin 2 and purified pregnancy associated haptoglogin (Hpr), as well as the corresponding immunoblots with anti-haptoglobin, and anti-PAPP-A.

Figure 5 shows tryptic peptide maps of haptoglobin alpha chains of haptoglobin 1, haptoglobin related protein (Hpr), haptoglobin 2, and a mixture of haptoglobins.

Figure 6 shows polyacrylamide gel electrophoresis of haptoglobin 1 and haptoglobin related protein. Panels B-E are immunoblots of the same proteins with various antibody preparations.

Figure 7, Panel A shows immunohistochemical staining of human breast cancer cells using antibodies raised against a synthetic peptide whose sequence corresponds to the the hpr gene. Panel B shows the same experiment except that the antibody was preincubated with purified Hpr from pregnancy serum. Panel C shows staining with anti-CEA, which was not abolished by preincubation with Hpr (Panel D).

Figure 8 shows the correlation of cancer related haptoglobin staining with relapse of breast cancer.

Figure 9 shows the staining of a breast cancer cell line With antibody raised against a synthetic peptide made according to the sequence of the hpr gene.

Figure 10 shows the staining of decidua with antibodies raised against the synthetic peptide made according to the predicted sequence from the hpr gene.

Figure 11 depicts a western blot analysis of reactive proteins in human decidua using antibodies raised against the synthetic peptide made according to the predicted sequence from the hpr gene.

### DETAILED DESCRIPTION OF THE INVENTION

It is a finding of the present invention that Hpr protein, which has hitherto been undetected, can be found in various human cells and tissues. These include: human breast carcinoma tissue, human decidua and placenta, serum from pregnant women, and cultured human breast carcinoma cell lines. Hpr protein from pregnancy serum has been purified using physicochemical methods to be substantially pure of Hp1 and Hp2. Hpr protein, as predicted from its nucleotide sequence, is very similar to haptoglobins 1 and 2, although there are a number of amino acid differences which cluster at the N-terminus of the alpha chain.

A synthetic polypeptide has been made having the following amino acid sequence: leu-tyr-ser-gly-asn-asp-val-thr-asp-ile-ser-asp-asp-arg-phe-pro-lys-pro-pro-glu-ile-ala-asn-gly-tyr-val-glu-lys-leu-phe-arg-tyr-gln-cys. This polypeptide generally corresponds to the 34 N-terminal amino acids predicted from the nucleotide sequence of the Hpr gene as identified by Maeda. This peptide is able to stimulate the production of antibodies in a mammal which are immunoreactive with epitopes found on the Hpr gene product but which are not found on either haptoglobins 1 or 2. These antibodies can be used to reliably detect Hpr expression in any human tissue as well as to purify Hpr using immunoaffinity techniques, which are well known in the art.

The substantially pure preparation of polypeptide having an amino acid sequence corresponding to the nucleotide sequence of the hpr gene can be made using any of the techniques which are known in the art. For example, the Merrifield technique (Journal of American Chemical Society, vol. 85, pp. 2149-2154, 1968), can be used. Substantial purity means that the preparation is almost totally free of haptoglobins 1 and 2. Although a sequence which corresponds to the 34 N-terminal amino acids of the predicted Hpr protein has been used, other polypeptides can be used as well. Other polypeptides may be longer or shorter or have conservative amino acid changes which do not change the epitope(s) found on Hpr but not found on Hp1 or Ep2. Polypeptides are designed by comparing the amino acid sequences of Hpr, haptoglobin 1 and haptoglobin 2, and utilizing those regions of sequence which have the maximum amount of differences. The Hpr sequence disclosed in the publication of Maeda, Journal of Biological Chemistry, vol. 260. pp. 6698-6709, 1985 is listed in Table I given below, and the haptoglobin 1 and 2 sequences can be obtained from the database of the National Biomedical Research Foundation as well as from Kurosky, Proceedings of a National Academy of Sciences USA, vol. 77, pp. 3388-3392, 1980; Black et al., Nature, vol. 218, pp. 736-741, 1968; Black et al. Canadian Journal of Biochemistry, vol. 48, pp. 123-132,

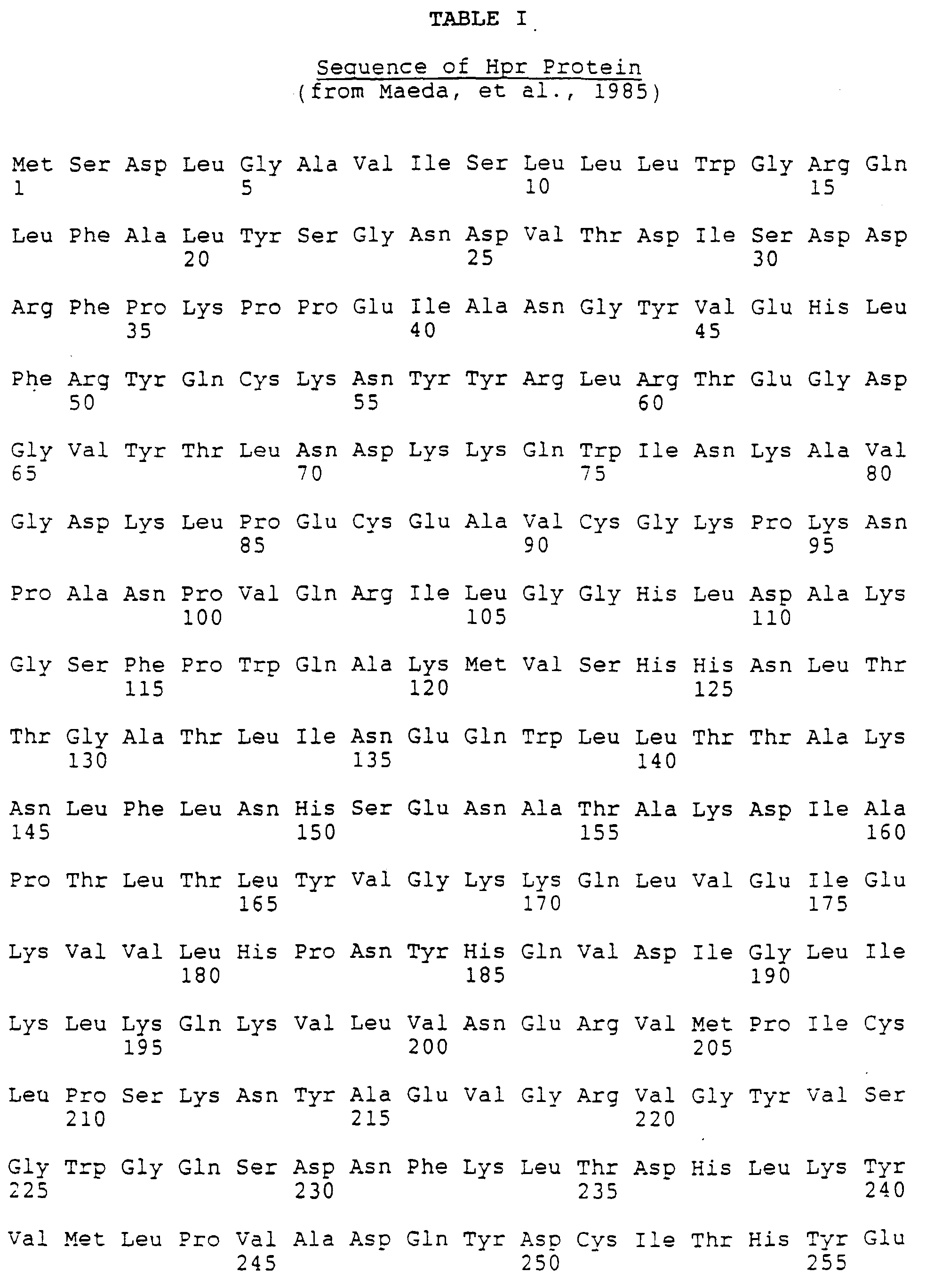

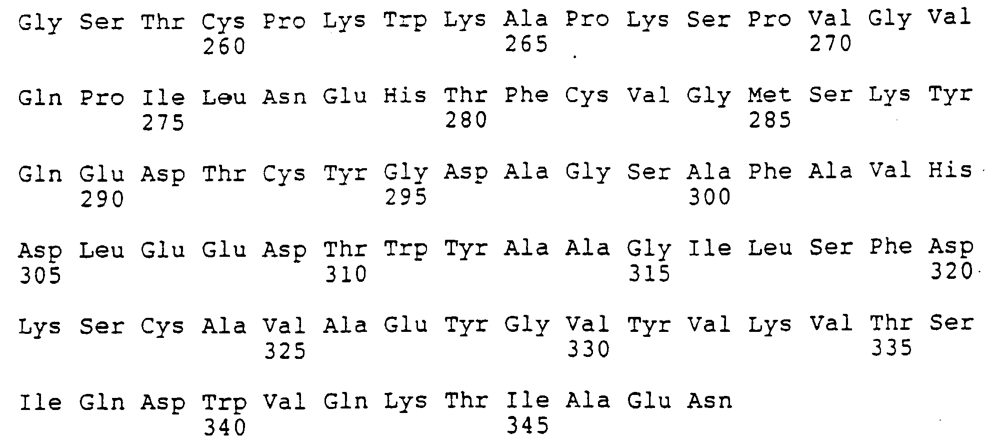

1970; and Black et al. Canadian Journal of Biochemistry, vol. 48, pp. 133-146, 1970. Polypeptides can be tested to determine if they are able to stimulate mammals to produce antibodies which are immunoreactive with epitopes found on Hpr but not found on Hp1 or Hp2. Methods of immunizing mammals to stimulate antibody production are well known in the art. Methods for testing the immunoreactivity of antibodies for known antigens are also well known.

The substantially pure preparation of polypeptide of the present invention can be used to affinity purify antibodies specific for the Hpr protein. In addition, the preparation of polypeptide of the present invention can be used to stimulate production of antibodies in a mammal by immunizing the mammal with the preparation. Usually, such immunization will employ coupling of the polypeptide to a larger immunogenic substance such as keyhole limpet hemocyanin. For affinity purification purposes, the polypeptide can be coupled to an inert matrix, such as agarose beads. Techniques for such coupling are well known in the art. The preparation of the polypeptide can also be used to quantitate Hpr-specific antibodies in an antibody preparation. In such a case, the synthetic peptide will usually be coupled to a larger inert proteinaceous substance such as bovine serum albumin. Once again, the techniques for coupling polypeptides to such matrices are well known in the art.

As mentioned above, antibodies which are specific for the Hpr protein in that they are immunoreactive with Hpr protein but not with either haptoglobins 1 or 2 can be made using a synthetic polypeptide of the present invention. Immunization of mammals such as rabbits, mice, goats, etc. to produce antibodies is well known in the art. Such polyclonal antibody preparations can be partially purified using immunoaffinity techniques employing a synthetic polypeptide of the present invention. Such purification methods are well-known in the art. Monoclonal antibodies can also be raised which are specific for Hpr epitopes and do not cross-react with haptoglobin 1 or 2. Generally, a rat or mouse will be immunized with the synthetic polypeptide (or Hpr protein) of the present invention and the rodent will later be sacrificed and spleen cells recovered for fusion with myeloma cells. Hybrid cells can be selected according to techniques known in the art, for example, selections involving complementation of two phenotypes, one from each parental cell. Antibody production of each hybrid cell can be screened individually. Antibodies which bind to epitopes on Hpr but not on Hp1 or Hp2 may bind Hpr or any other protein expressing these epitopes.

In order to screen for antibodies which are immunoreactive with epitopes found on the Hpr gene product but not found on Hp1 or Hp2, a simple battery of tests can be performed. Antibodies can be tested for immunoreactivity with Hpr using a substantially pure preparation of Hpr, or with the preparation of polypeptide of the present invention conjugated to a larger moiety such as bovine serum albumin. The desired specific antibodies should be positive in either or both of these tests. The antibodies should also be tested for immunoreactivity with Hp1 and Hp2. Desired antibodies having absolute specificity for Hpr should be negative in both such tests. Antibodies can also be detected using this battery of test which have relative specificity for Hpr relative to Hp1 and Hp2. That is, some monoclonal antibodies can be found which react more strongly with Hpr than with Hp1 and Hp2. These antibodies of relative specificity for Hpr may also be useful. Means for using them are discussed below.

Immunoaffinity techniques to purify monospecific polyclonal antibodies reactive with Hpr but not with Hp1 or Hp2 can be used. Similar binding properties are employed as in the tests described for monoclonal antibodies above. That is to say that antibodies which immunoreact with Hpr will be positively selected, while those that immunoreact with Hp1 and Hp2 will be removed from the desired antibody preparation.

Antibodies which show relative or preferential specificity for Hpr relative to Hp1 and Hp2 can be rendered absolutely specific by altering the conditions under which immunoreactivity is assayed. Conditions in the assay medium which can be altered include, but are not limited to: the ionic strength; the detergent concentration; the concentration of chaotropic agents, such as urea, guanidine, and potassium thiocyanate; and the pH. Alteration of these conditions leads to destabilization of the various bonding forces which contribute to antibody-antigen binding. Titration of reagents altering each of these conditions allows determination of a set of conditions where relatively or preferentially specific antibodies immunoreact with Hpr but not with Hp1 or Hp2. Suitable ranges in which to vary the destabilizing agent concentrations can readily be determined. For example, in order to alter ionic strength, potassium chloride can be titrated from about 0.05M to 2M. Detergents, either ionic or non-ionic, can be titrated from about 0.05% to 2%. Chaotropic agents can be titrated from about 0.5M to 8M. The range of pH can be titrated from about 2 to 10. Such conditions can be useful both to screen for monoclonal antibodies immunoreactive with Hpr and to assay for Hpr in various biological sources. The above-described method of titrating various destabilizing agents can also be used with other antibody-antigen pairs to enhance the specificity of reaction.

Applicants have found Hpr in four different human cell types. Three of these cell types can practically be used to produce amounts of Hpr for biochemical studies as well as studies to determine the biological effect of Hpr under physiological conditions. One particularly useful source is human breast carcinoma cell lines, which can be obtained from the American Type Culture Collection in Rockville, Maryland. One such cell line is called MDA-MB-231, and is an estrogen receptor negative cell line. Hpr epitopes have been detected in the cytoplasm of about 50% of such breast carcinoma cell lines. Those cell lines readily can be indentified, e.g., by use of antibodies immunoreactive with the 34 amino acid polypeptide described above.

One means of preparing an Hpr preparation is to culture the human breast carcinoma cells which produce Hpr protein using culture conditions which are well-known in the art. The cells are then harvested and the cell membrane disrupted to obtain a cell lysate. The nuclei and membrane fractions can be removed and a cytoplasmic fraction containing Hpr is obtained. Means of separating various cellular fractions, such as differential centrifugation, are indeed well-known in the art.

Another source of Hpr protein has been found to be human decidua tissue and placenta. Such tissue can be collected from different sources and homogenized according to techniques well-known in the art. The cell membrane of the homogenized cells of the tissue can then be lysed to obtain a cytoplasmic fraction containing Hpr protein. Serum from pregnant women has also been shown to contain Hpr in recoverable amounts. Similarly, histological sections of breast tumors contain Hpr protein, but this is a difficult tissue source to obtain in a quantity and manner permitting purification of Hpr.

Once a cytoplasmic fraction containing Hpr is obtained, purification of Hpr can be accomplished according to techniques which are well-known in the protein purification art. For example, various types of chromatography may be used. Columns which have been found to be particularly useful include a Cibacron F3GA Sepharose column, a DEAE cellulose column, an anion exchange column, as well as a gel permeation column.

Hpr can also be purified using immunoaffinity techniques. As antibodies are provided here which are specific for Hpr epitopes, Hpr protein can be positively selected from a mixture of many proteins. The use of the antibodies of the present invention to purify the protein allows good separation from the proteins which are most similar to Hpr, namely haptoglobins 1 and 2. Of course, other techniques of purification are known in the art and can be used to purify Hpr.

The Hpr preparations which are obtained according to the present invention are substantially pure and probably homogeneous. This conclusion is based on visualization of proteins electrophoretically separated on polyacrylamide gels in which no Hp2 is detected. Generally, substantially pure preparations are free of Hp1 and Hp2 and will have greater than 75% of the haptoglobin type protein present being Hpr protein. More preferably, the preparations will contain greater than 90% of the haptoglobin type protein as Hpr.

The antibodies of the present invention can be used to detect Hpr epitopes in histological sections of breast cancer tissue as well as in other solid tumors such as, lung cancer tissue, genito-urinary tumor tissue and gastrointestinal tumor tissue. It has been found that the presence of Hpr epitopes in breast cancer tissue correlates with a worsened prognosis; it indicates that the cancer will probably recur early and metastasize early, compared to those whose tissues are Hpr negative. Breast tissues in which Hpr epitopes are found are characterized by three qualities: the immunoreactivity is present in infiltrating breast carcinoma; granular cytoplasmic immunoreactivity is observed without nuclear staining; and the staining is heterogeneous, i.e., there is cell-to-cell or region-to-region variability.

One can detect antibody binding to tissue sections by any detection means known in the art for example, a radiolabel or a stain. A particularly useful stain employs peroxidase, hydrogen peroxide and a chromogenic substance such as aminoethyl carbazole. The peroxidase (a well known enzyme available from many sources) can be coupled to the anti-Hpr antibody or merely complexed to it via one or more antibodies. For example, a goat anti-peroxidase antibody and a goat anti-Hpr antibody can be complexed via an anti-goat IgG. Such techniques are well known in the art. Other chromogenic substances and enzymes may also be used. Radiolabeling of antibodies may also be used to detect antibody binding to sections. Labeled antibodies may be anti-Hpr or second antibodies immunoreactive with anti-Hpr antibodies. Again, such techniques are well known. The precise technique by which Hpr is detected in breast cancer patients is not critical to the invention. Biochemical or immunological techniques can now be used which do not employ immunohistochemistry, although that is the preferred method of the present invention.

Hpr can be quantitated in a biological fluid, such as serum, plasma, effusions, ascites, urine cerebrospinal fluid, and fluids of ovarian origin, using any protein detection means known in the art. Preferred methods employ immunological detection means. These include: radioimmunoassay, enzyme linked immunoadsorbent assay, complement fixation, nephelometric assay, immunodiffusion or immunoelectrophoretic array. Plasma should be anti-coagulated before use, as is known in the art. Cellular elements and lipid should be removed from fluids, e.g., by centrifugation. For dilute fluids, such as urine, protein should be concentrated, e.g., by ultra-filtration or salting-out.

One particularly preferred method of detecting and/or quantitating Hpr in fluid samples employs a competitive assay. An antibody immunoreactive with an epitope found on Hpr but not found on Hp1 and Hp2 is attached to a solid support such as a polystyrene microtiter dish or nitrocellulose paper, using techniques known in the art. The solid support is then incubated in the presence of the fluid to be analyzed under conditions where antibody-antigen complexes form and are stable. Excess and unbound components of the fluid are removed and the solid support is washed so that antibody-antigen complexes are retained on the solid support. A fixed amount of a polypeptide containing an epitope found on Hpr but not found on Hp1 or Hp2, is then incubated with the solid support. The polypeptide binds to an antibody immunoreactive with Hpr which is attached to the solid support. The polypeptide has been conjugated to a detectable moiety, such as biotin, peroxidase or radiolabel, by means well known in the art. Excess and unbound polypeptide is removed and the solid support is washed, as above. The detectable moiety attached to the solid support is quantitated. Since the Hpr and the polypeptide have competed for the same antibody binding sites, Hpr in the fluid can be quantitated by its diminution of the binding of the polypeptide to the solid support. Antibodies employed in this assay may be immunoreactive with Hpr out not with Hp1 or Hp2. Alternatively, relatively specific antibodies may be used under conditions which destabilize immunoreactivity with Hp1 and Hp2. Polyclonal antibodies which contain an antibody species immunoreactive with an epitope on Hpr but not on Hp1 or Hp2, may also be used.

Although this assay has been described with particularity to the Hpr system, it can also be used to quantitate other protein analytes in biological fluids. That is, a detectably labeled polypeptide which shares an epitope with a protein analyte can be used to quantitate the analyte. A monospecific antibody is not required as the specificity is provided to the assay by means of the polypeptide.

Elevated levels of Hpr in the blood or other biological fluid from a patient correlates with proliferation and likely metastasis of breast cancer as well as other solid tumors. Other tumors include lung cancer, genito-urinary tumors, and gastrointestinal tumors. The determination of elevated levels of Hpr is done relative to a patient with no detectable solid tumor. This may be the same patient or a different patient. For example, a first sample may be collected immediately following surgical removal of a solid tumor. Subsequent samples may be taken to monitor recurrence of tumor growth and/or tumor cell proliferation. In addition, the assay of Hpr in biological fluids can be used to distinguish between neoplastic and non-neoplastic fluid accumulations in patients carrying a malignant diagnosis.

The following examples are not intended to limit the invention, but merely provide specific embodiments which can be employed.

### Example 1

This example describes the isolation and purification of Hpr protein from human pregnancy plasma, and the separation of the two subunits.

Maternal plasma from third trimester pregnancies was obtained from discarded EDTA-anticoagulated whole blood samples. The plasma was stored at -70°C until use.

Proteins reactive with the whole IgG fraction of a rabbit antiserum to pregnancy-associated plasma protein A (PAPP-A) (Dako Laboratories, Santa Barbara, Ca.) were purified by successive chromatographic steps. The purification was monitored by optical density of chromatographic effluents at 280 nm, and analysis of fractions using Coomassie-stained Laemmli gels, and Western blotting. Initially, 80 ml of plasma was loaded onto a 2.5 x 30 cm Cibacron Blue F3G-A Sepharose (trade mark) (Pharmacia) column equilibrated in 50 mM sodium phosphate pH 6.8 at 4°C. The flow-through containing all the detectable immunoreactive material was dialyzed against 20 mM sodium phosphate pH at 6.8 at 4°C with a mM NaN₃, and applied to 2.5 x 10 cm column of DE-52 DEAE-cellulose (Pierce Chemical Co) equilibrated in the same buffer. The immunoreactive material was eluted with 0,2 M NaCl in the starting buffer. The eluate was dialyzed against 20 mM Tris-HCl, pH 8.5 at 4°C containing 0.15 M NaCl, Mm beta-mercaptoethanol, and 1 mM NaN₃, then loaded onto a 2.5 x 60 cm column of Fast Flow Q-Sepharose (Pharmacia, Piscataway, N.J) equilibrated in the same buffer. The column was eluted with a 500 ml linear gradient of 150 mM to 500 mM NaCl at a flow-rate of 100 ml/hr at 4°C. The immunoreactive protein species eluted in the range between 0.25 and 0.28 M NaCl. The fractions containing immunoreactive protein were pooled and dialyzed against 200 mM Tris-acetate, pH 7.5 at 4°C with 1 mM beta-mercaptoethanol and 1 mM NaN₃ and applied to a 5 x 90 cm Sepharose CL-4B (Pharmacia) column.

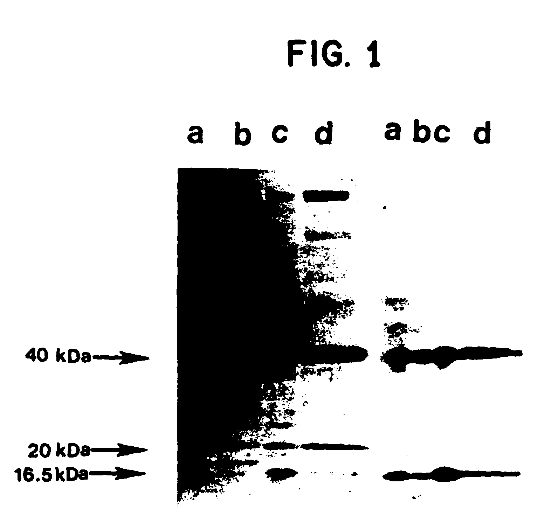

Figure 1 summarizes the purification of the dominant immunoreactive bands recognised by the polyspecific anti-PAPP-A (Dako). The figure shows a Coomassie stained 10-15% Laemmli gel (Nature, vol 227, pp. 680-685, 1970) and corresponding Western blot.

Western blotting was carried out essentially according to published procedures. Following NaDodSo₄ polyacrylamide gel electrophoresis, protein was transferred to 0.45 u nitrocellulose sheets (Schleicher & Schuell) in 96 mM glycine, 12.5 mM Tris, 0.1% NaDodSO₄, and 20% methanol at 40 V, 250-300 mA, at 4°C for 6 hrs. After evaluating transfer efficiency by Ponceau S staining, the membranes were blocked with 3% bovine serum albumin, then incubated sequentially with anti-PAPP-A antibody for 2 h and protein A labeled with ¹²⁵I for 1 h (Gershoni, et al. Analyt. Biochem., vol. 131, pp. 1-15, 1983). Washes after each step were performed in Tris-saline containing 1 mM NaN₃.

Sequential chromatographic steps involved removal of albumin and additional protein species on Cibacron Blue F3GA Sepharose, (Lane a), step elution from a DEAE cellulose column (Lane b), gradient elution from a Fast-Flow Q Sepharose anion exchange column (Lane c), and gel permeation chromatography on Sepharose CL-4B (Lane d). Interestingly, gel filtration incompletely resolved the immunoreactive proteins into three consecutively-eluting species consisting of the 40 kDa species co-eluting with the weakly reactive 20 kDa species, the 40 kDa band co-eluting with a weakly immunoreactive 16.5 kDa species, and lastly, as a separate, albeit overlapping peak, the 40 kDa species co-eluting with strongly immunoreactive 16.5 kDa band.

To separate the individual immunoreactive chains under denaturing conditions, fractions containing immunoreactive protein were pooled and dialyzed against 20 mM Tris-HCl, 6 M urea, 10 mM beta-mercaptoethanol, pH 8.5 at 25°C (dialysis was performed at 4°C, but the pH was adjusted to be 8.5 at the indicated temperature). The final purification step utilized HPLC on a Waters system with a 5 x 50 mm Mono Q HR5/5 (Pharmacia) column monitored at 280 nm. The sample was injected in the Tris-urea-mercaptoethanol starting buffer and following 10 ml of isocratic flow at 2 ml/min, a linear gradient of 30 ml over 15 min was applied to final conditions of starting buffer with 0.5 M NaCl.

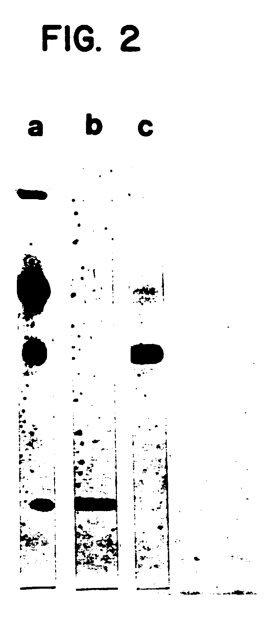

A Coomassie blue stained 10% Laemmli gel showing the separation of immunoreactive heavy and light chains by gradient anion exchange chromatography on Mono Q HR5/5 using HPLC is shown in Figure 2. Lane a is 100 ug of purified immunoreactive protein. Lane b contains the alpha chains, which flowed through in the void volume, while lane c contains the immunoreactive 40 kDa chain which eluted in 0.85 M NaCl. Fractions containing the 40 kDa species were pooled, dialyzed against 0.2 M NH₃HCO₃ buffer and lyophilized.

### Example 2

This example demonstrates that a 20-amino acid stretch of the heavy chain (beta chain) of Hpr is 100% homologous to the beta chains of haptoglobin 1 and 2.

The lyophilized samples of the 40 kDa species made according to Example 1 were dialyzed against three changes of 0.2 M NH₄HCO₃ and lyophilized, then thrice re-lyophilized form HPLC-grade water. Gas phase sequencing was carried out. Samples of intact protein chains generally consisted of 100 ug of protein as determined by Lowry assay (J. Biol. Chem., vol. 193, pp 265-275, 1951). Peptide samples generally consisted of 0.5 - 2 nanomoles as quantitated by the sequencer. Protein was sequenced on an Applied Biosystems Model 470A Sequenator equipped with on-line phenylthiohydantoin (PTH) analysis using the regular program O3RPTH. The PTH derivatives were separated by reversed phase HPLC over an Applied Biosystem 200 x 21 mm C-18 column.

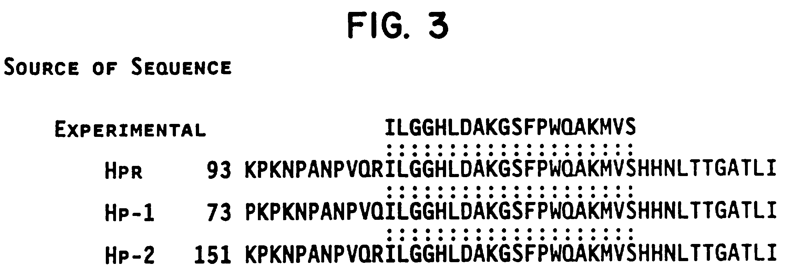

N-terminal sequencing yielded twenty residues shown in Figure 3. Using the protein sequence database of the National Biomedical Research Foundation and the DFASTP alignment program three protein sequences showed 100% homology over the entire 20 amino acid stretch: the beta-chain of human haptoglobin 1; the beta-chain of human haptoglobin 2; and the predicted beta-chain of haptoglobin-related protein precursor.

### Example 3

This example demonstrates that the haptoglobin isolated from pregnancy serum according to Example 1 differs from Hp1 and 2 in its 16.5 kDa chain.

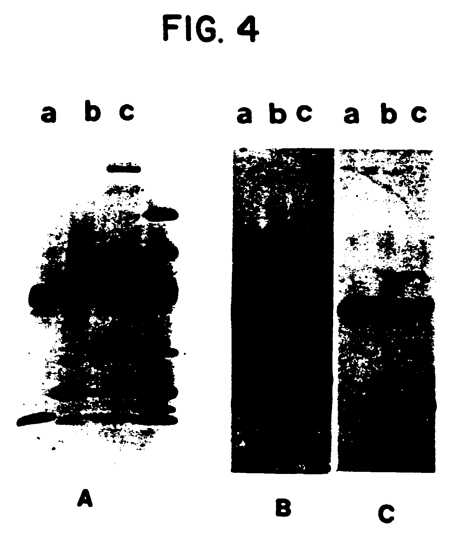

Although sequence analysis placed the pregnancy plasma protein unequivocally in the haptoglobin (Hp) gene family, more specific assignments could not be made, since the N-terminal sequence for the beta-chains of all species is identical. Figure 4 shows the results of an immunologic analysis performed with purified Hp1 and 2 standards, the haptoglobin purified from pregnancy serum, rabbit anti-haptoglobin and the anti-PAPP-A. Panel A shows a 10-15% Coomassie stained Laemmli gel of Hp1, Hp2, and the haptoglobin purified from pregnancy serum (lanes a-c, respectively). Panel B is a corresponding immunoblot with anti-haptoglobin, 1:50; panel C is an immunoblot with anti-PAPP-A, 1:50.

Anti-haptoglobin reacts strongly with all the 40 kDa beta chains, and Hp1 and 2 alpha chains; the 16.5 kDa band from lane c containing the purified protein is only weakly reactive. In contrast, anti-PAPP-A, while strongly labeling the 40 kDa beta chains, reacts weakly with Hp2 alpha chain and is only slightly reactive with Hp1 alpha chain. The 16.5 kDa band from the purified protein in lane C is disproportionately intense in comparison to its Coomassie staining, particularly when compared to the disproportionately weak relative immunoreactivity of the Hp1 alpha chain shown in lane a. Thus, the alpha chain isolated from pregnancy plasma is immunologically distinct, containing epitopes seen by the anti-PAPP-A which are not present in either the Hp1 or Hp2 alpha chains.

### Example 4

This example demonstrates that there are sequence differences between the alpha chain of Hpr and those of Hp1 and Hp2. In addition, the difference seen is consistent with the assignment of the haptoglobin from pregnancy serum as being the hpr gene product.

Peptide mapping was performed using the Elder technique (Speicher, et al. Proc. Natl. Acad. Sci. U.S.A., vol 77, pp 5673-5677, 1980; and Elder, et al., J. Biol. Chem., Vol. 252, pp 6510-6515, 1977). Briefly, slices containing the desired proteins were exercised from Coomassie-stained gels, exhaustively radiolodinated, and enzymatically digested with trypsin. The resultant limit peptides were then subjected to high-voltage electrophoresis along one dimension of a cellulose sheet, followed by ascending partition thin layer chromatography in the perpendicular dimension.

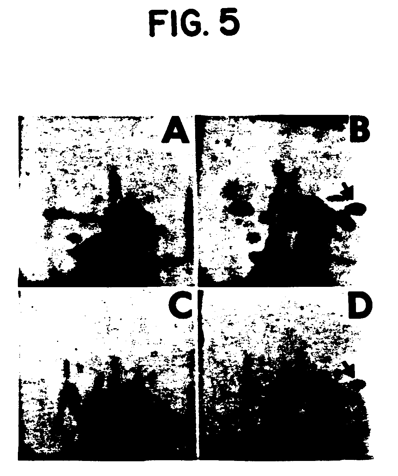

Peptide mapping by this technique detects only tyrosine-containing peptides. Using the published amino acid sequences, the family of tryptic peptides from the chains of Hp1 and Hp2 should each be similar, since Hp2 resulted from a partial reduplication of Hp 1 (Figures 5a and 5c). The principal differences in amino acid sequence between Hpr, Hp1 and Hp2 lie in the alpha chains. Within the alpha chains, these differences cluster principally at the N-termini. With reference to peptide mapping, using the deduced Hpr alpha chain sequence one would predict the same tryptic peptides as obtained from Hp1 plus one additional tyrosine-containing peptide. Figure 5b shows the peptide map of the purified protein alpha chain with an arrow highlighting the one additional peptide obtained experimentally. These results thus suggest that the unique haptoglobin purified from pregnancy plasma is the Hpr gene product. Figure 5d is a mixing experiment where Hp1 and the purified protein alpha chain were mixed before mapping. Again, the additional peptide is present but with less relative intensity, while the shared peptides all co-migrate.

### Example 5

This example demonstrates that the alpha chain of the haptoglobin from pregnancy serum shares epitopes with a synthetic peptide made according to the deduced Hpr sequence.

A synthetic peptide corresponding to the 34 N-terminal residues of the predicted Hpr gene product (Maeda et al. Proc. Natl. Acad. Sci. USA, vol. 83 pp.7395-7399, 1986) was synthesized on an Applied Biosystems Model 43A peptide synthesizer by the solid phase method of Merrifield (J. Am. Chem. Soc., vol. 85, pp. 2149-2154, 1968). Because histidine coupling can be inefficient, lysine was conservatively substituted for the histidine in position 28.

Two separate columns were made using beads coupled to: haptoglobin 1, and the 34-mer synthetic peptide. Haptoglobin 1 (Hp1) and the Hpr synthetic peptide were immobilized using agarose beads derivatized with 1,1-carbonyldimidazole (Reacti-Gel 6X, Pierce) which results in a stable N-alkylcarbamate linkage. 5ml of gel was washed with 0.1 M borate and 0.15 M NaCl, pH 8.5, RT to remove the acetone. 4 mg of Hp1 (Sigma) diluted to 1 mg/ml in borate buffer, and 50 mg Hpr synthetic peptide diluted to 12.5 mg/ml in borate buffer were each added to 5 ml of gel and incubated with agitation at 4°C for 30 hr. After incubation, the supernate was decanted and an equal volume of 0.2 M Tris was added to quench any remaining reactivity. The gels were then extensively washed with Tris-saline buffer. The Hpr peptide column was then denatured with 100 ml of 0.1% NaDodSO₄ in Tris-saline buffer.

Anti-Hpr antibodies reactive with the Hpr peptide were isolated from the crude anti-PAPP-A serum by means of sequential affinity chromatographic steps. First, 100 ul of crude anti-PAPP-A antibody in 10 ml Tris-saline buffer was incubated with the Hp1 gel overnight at 4°C with agitation. The gel was next washed extensively with Tris-saline and the flow-through collected over a 5 ml hydroxyapatite column. Antibodies reactive with the Hp1 column were eluted with 20 ml of acetate buffer (0.05 M sodium acetate, 0.15 M NaCl, pH 3.5, at room temperature). The antibodies which flowed through the column were eluted from the hydroxyapatite column with 20 ml of 0.4 M sodium phosphate. Both sets of antibodies were dialyzed against 2 l of Tris-saline buffer.

The antibodies unreactive with the Hp1 column were then incubated with the denatured Hpr peptide gel overnight at 4°C with agitation. Antibodies reactive with the Hpr peptide were eluted with 20 ml of acetate buffer and dialyzed against Tris-saline. Each of these antibody fractions were used to label immunoblots of 10-15% gradient Laemmli gels on which the subunits of haptoglobin 1 and the haptoglobin related protein (according to example 1) were separated by electrophoresis.

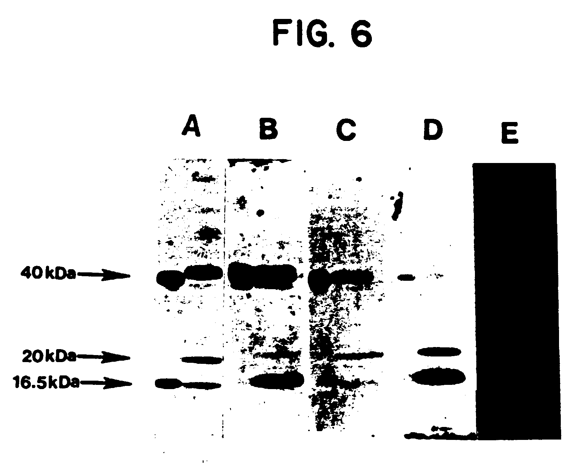

Figure 6, panel A shows a Coomassie stained 10-15% gradient Laemmli gel of 25 ug Hp1 in the left lane, and 100 ug haptoglobin related protein on the right. Panels B-E are immunoblots of the same protein loads. Panel B was incubated with unabsorbed anti-PAPP-A antibody, 1:50. Panel C was incubated with the subset of anti-PAPP-A antibodies which bound to immobilized Hp1; panel D was incubated with the unreactive antibodies which flowed through. Note the enrichment for antibodies to the 40 kDa beta chains which bound to the Hp1 gel in panel C; while antibodies to the unique immunoreactive alpha-chain were unreactive with immobilized Hp 1 and flowed through (panel D). Panel E was incubated with the subset of antibodies seen in panel D which reacted with immobilized, denatured, synthetic Hpr peptide. Note that these antibodies, which bound to the synthetic Hpr peptide, react only with the 16.5 kDa alpha chain of the pregnancy associated haptoglobin. This demonstrates that the unique alpha-chain of this haptoglobin contains Hpr epitopes.

### Example 6

This example shows that Hpr epitopes are identified in the cytoplasm of human breast carcinoma.

Paraffin embedded human breast carcinoma specimens were obtained from the files of the Department of Pathology of the Johns Hopkins Hospital. Buffered 10% formalin-fixed, parrafin embedded 6 u tissue sections were deparaffinized in xylene, and rehydrated in alcohol-water baths, then incubated in Tris-saline at pH 7.6, followed by blocking of endogenous peroxidase activity with 3% H₂O₂ for 15 min. After Tris-saline washes, sections were blocked with normal swine serum diluted 1/100 (Dako) for 30 min, followed by incubation with specific antibody or non-immune serum overnight at 4°C. After washing in Tris-saline, swine-anti-rabbit immunoglobin IgG fraction (Dako), 1/100 in 0.5 M tris buffer was applied for 30 min, followed by a peroxidase-rabbit-anti-peroxidase complex (Dako). After final washing, the slides were incubated with diaminobenzidine (Sigma) substrate for 6 min, counterstained in Mayer's hematoxylin, coverslipped, and examined.

Antibodies were raised against Hpr by immunization of rabbits with the 34-mer synthetic peptide described above in Example 5. For purposes of immunization, the peptide was conjugated to keyhole limpet hemocyanin (KLH) (Boehringer Mannheim) essentially as described (Lerner, et al., Proc. Natl. Acad. Sci. USA, vol. 78, pp. 3403-3407, 1981). 8 ul of an 8.75 mM solution of malemidobenzoyl-n-hydroxy-succinimide (MBS) (Pierce Chemical Co.) in dimethyl formamide were added to 14.8 mg of KLH in phosphate-buffered saline and incubated at room temperature for 30 min. The KLH-MBS conjugate was separated from unreacted MBS by gel filtration on a Pharmacia PD-10 column. 1.2 mg of Hpr peptide was added directly to the KLH-MBS and mixture incubated a further 30 min at room temperature. To estimate coupling efficiency, a 35 ul sample of the unfractionated 3.0 ml reaction volume was chromatographed on a Pharmacia Superose 12 column using a Waters HPLC at 1 ml/min in PBS and the effluent monitored at 214 nm. Unreacted peptide was quantified by peak integration and comparison to standards of peptide run alone. This method yielded an approximate substitution ratio of 4.6 peptides per KLH molecule.

Two Pasteurella-free New Zealand White rabbits (Dutchland) were each inoculated on Day 0 with 200 ug of antigen in 1 ml of an emulsion comprised of equal parts of phosphate buffers saline (PBS) and complete Freund's adjuvant; the inoculae were divided among 3-4 subcutaneous and intramuscular sites. On Day 14, the animals were boosted with similar amounts of antigen in incomplete Freund's adjuvant. The rabbits were test bled on Day 32; serum from the positive rabbit was collected over the ensuing month.

Bovine serum albumin was derivatized with the synthetic Hpr peptide as described above. An enzyme-linked immunosorbent assay was performed as described by (Voller et al., J. Clin. Pathol., vol. 31, pp. 507-520, 1978) using 2 ug of BSA-peptide per well which was tested against dilutions of rabbit serum and developed with protein A-horseradish peroxidase conjugate and o-phenylene diamine substrate.

To purify the antibody, a 10 cm x 5 mm Selectispher-10 activated tresyl column HPLC affinity column (Pierce Chemical Co.) was derivatized with the Hpr synthetic peptide. The column was washed with 30 ml PBS at a flow rate of 2 ml/min, then 27 mg of peptide dissolved in 4.5 ml of PBS was passed through the column at a flow rate of 1 ml/min. Unreacted tresyl groups were capped by passing 30 ml of 0.2 M Tris HCl, pH 8.0 through the column. For antibody purification, the column was equilibrated in 300 mM NaCl, 20 mM sodium phosphate, pH 7.5 at room temperature. 10-20 ml serum were loaded onto the column at a flow rate of 0.5 ml/min. After loading, the column was washed with buffer until the optical density of the effluent at 280 nm returned to zero. Antibody was then eluted with 6 M urea in the same buffer, and the antibody-containing fractions immediately dialyzed into Tris-buffered saline containing 1 mM NaN₃.

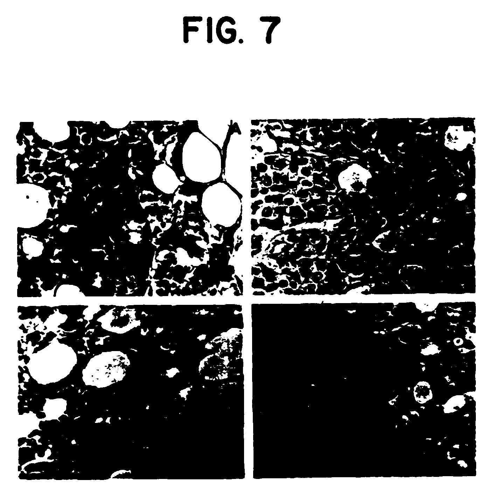

Figure 7 illustrates immunoperoxidase staining of a primary breast adenocarcinoma with both anti-Hpr antibody (Panel 7a) and anti-CEA antibody (Panel 7c); note the intense cytoplasmic positivity of anti-Hpr staining. When anti-Hpr antibody was incubated for 2 hr at 4°C with native Hpr, positive cytoplasmic staining was abolished (Panel 7b). To establish specifity of the immunoabsorption, anti-carcinoembryonic antigen (CEA) antibodies were incubated under similar conditions with native Hpr. The cytoplasmic CEA positivity was not abolished by such pre-incubation with Hpr (Panel 7d). Together with the failure of Hp1 and Hp2 to inhibit staining, this establishes that epitopes derived from the N-terminus of the predicted alpha chain of the Hpr gene product can be detected in human breast carcinoma. Thus Hpr, a modified form of Hpr, or a highly cross-reactive protein must be present in human breast carcinoma cells.

### Example 7

This example demonstrates that there is a correlation between expression of Hpr by human breast cancer and the time interval that a patient remains disease-free.

Sixty-one cases of human breast cancer were available for concurrent evaluation of cancer-related haptoglobin expression and immunoreactivity with anti-PAPP-A antiserum. Cancer-related haptoglobin (Hpr) expression was evaluated by immunohistochemical staining of paraffin-embedded material using affinity-purified polyclonal antibody to a synthetic peptide whose sequence was derived from the cancer-related haptoglobin sequence. PAPP-A positivity was similarly evaluated using anti-PAPP-A antiserum. Of the 61 patients, 40 had Stage I disease (tumor less than 5 cm, negative lymph nodes) and 21 had Stage II disease (tumor less than 5 cm, positive axillary lymph nodes). Data were analyzed in a life table format using the BMDP Statistical Program. Two statistical tests, the Generalized Wilcoxon (Breslow), and the Generalized Savage (Mantel-Cox) were used to determine the significance of the differences between curves.

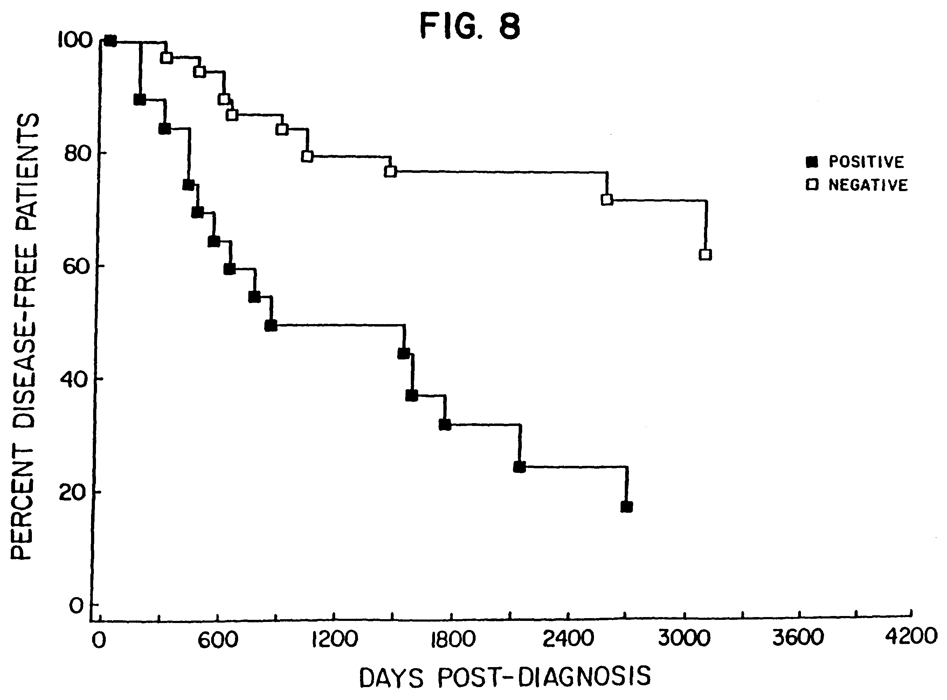

The results are shown in Figure 8. The ordinate shows the percentage of patients remaining disease-free, while the abscissa shows the time interval since diagnosis. The differences between the curves are highly significant, with p less than .0005 (Wilcoxon) and p less than .0003 (Savage).

### Example 8

This example demonstrates that cultured breast cancer cells express Hpr.

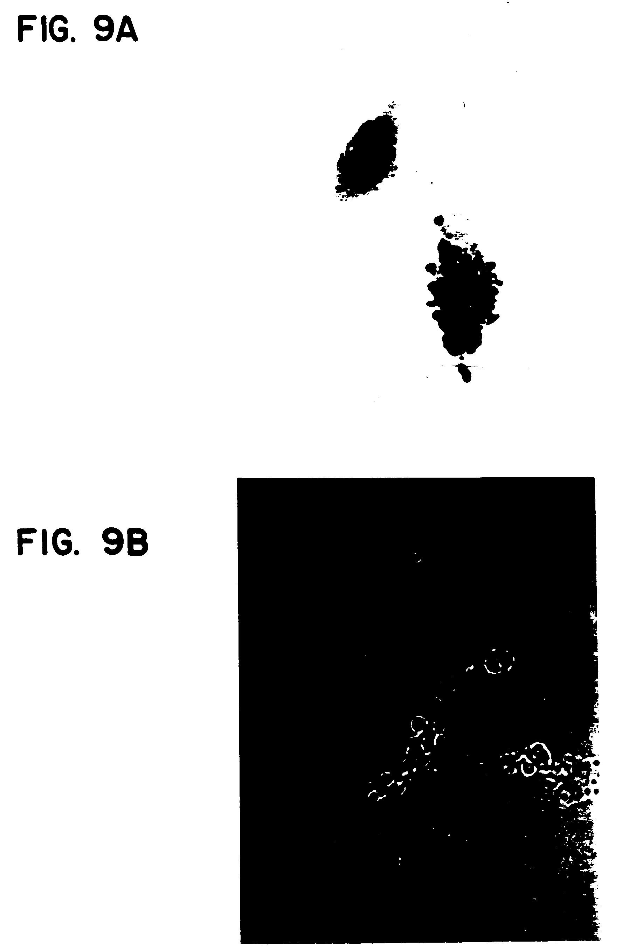

Although fresh human breast carcinoma can be used as a source of Hpr protein the quantities are generally small, and availability is irregular. For these reasons, a series of human breast carcinoma cell lines were obtained from the American Type Culture Collection (Rockville, MD) and screened for Hpr expression by an innunocytochemical procedure. Figure 9 shows the results of this approach in one such line, MDA-MB-231, an estrogen receptor negative cell line (Anderson, J. Submicroscop. Cytol. vol. 16, pp 673-690, 1984).

Briefly, cells were grown on chamber slides in L-15 medium supplemented with 10% fetal bovine serum and antibiotics, fixed in paraformaldehyde, permeabilized with Triton X-100®, incubated with anti-Hpr 40 ug/ml, or controls overnight at 4°C, and developed sequentially with a biotinylated secondary antibody, avidin-peroxidase, and aminoethyl-carbazole-peroxide substrate solution.

The left-hand panel shows granular cytoplasmic staining by the specific antibody, while the right-hand panel shows the control without primary antibody; unrelated affinity-purified antibodies stain with appropriate distributions when used as unrelated positive primary antibody controls in this method. Thus, it can be readily seen that these cultured cells can produce Hpr.

### Example 9

This example shows that decidua tissue synthesizes Hpr under physiologic conditions.

Because Hpr is purified from pregnancy serum, pregnancy-specific tissues were screened immunohistochemically for expression. Fresh decidua was obtained from tissues otherwise to be discarded following elective abortions and was formalin-fixed. Buffered 10% fomalin-fixed, parrafin embedded 6 u tissue sections were deparaffinized in xylene, and rehydrated in alcohol-water baths, then incubated in Tris-saline at pH 7.6, followed by blocking of endogenous peroxidase activity with 3% H₂O₂ for 15 min. After Tris-saline washes, sections were blocked with normal swine serum diluted 1/100 (Dako) for 30 min, followed by incubation with specific antibody made as described above in Example 6, or for control, with nonimmune serum overnight at 4°C and developed sequentially with a biotinylated secondary antibody, avidin-peroxidase, and aminoethyl-carbazole-peroxide substrate solution. The slides were incubated with diamino-benzidine (Sigma) substrate for 60 min, counterstained in Mayer's hematoxylin, coverslipped, and examined.

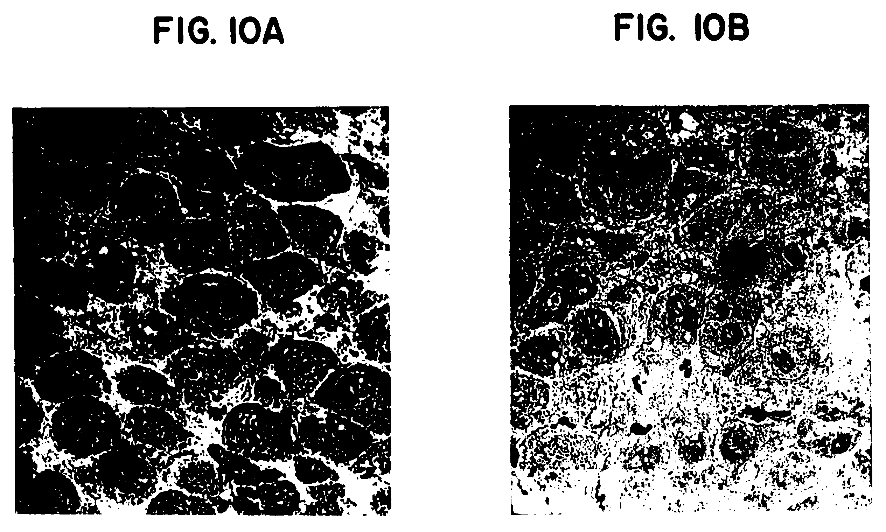

The result of decidual staining are seen in Figure 10. The left-hand panel shows the prominent cytoplasmic staining with the anti-Hpr antibody, while the right-hand panel shows a control where the primary antibody was omitted. Other placental tissues were negative (data not shown). This experiment suggests that decidua synthesizes Hpr under physiologic conditions.

### Example 11

This example demonstrates that the Hpr expressed by decidua is synthesized as a larger polypeptide than Hpr isolated from pregnancy serum.

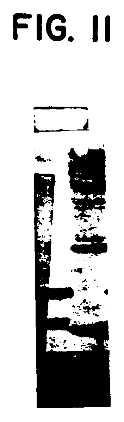

Figure 11 shows that anti-Hpr recognises an unexpectedly heavy alpha chain in lysates of decidua. Briefly, 1 f of frozen decidua was homogenized in 10 ml of lysis buffer (10 mM Tris HCl, pH 7.5 at 4°, 1 mM EDTA, 1% Triton X-100 (trade mark), 0.5 mM disopropylfluorophosphate) using a Brinkmann polytron. Insoluble material was removed by centrifugation at 1200 x g for 5 min at 4°C, and the resultant supernate mixed with Laemmli solubilizing buffer. A 50 ul aliquot was electrophoresed on a 10-15% polyacrylamide gel, then transferred to nitrocellulose using a semi-dry blotting apparatus (Polyblot, American Bionetics). The blot was blocked for 2 h at room temperature with 3% BSA in Tris-saline, then incubated overnight in anti-Hpr, 40 ug/ml in Tris-saline. Following incubation, the blot was then sequentially washed in PBS with 0.05% Triton-X-100®, incubated with biotinylated goat-anti-rabbit IgG, washed, incubated with avidin-horseradish peroxidase complex, washed, and incubated in aminoethylcarbazole substrate solution.

The blot shows that decidual lysate contains a unique alpha chain species. The left-hand lane shows a partially-purified Hpr preparation which is contaminated with haptoglobin 2; the anti-Hpr used in this experiment cross-reacts with haptoglobin 2 alpha chain, the uppermost band, but fails to react with haptoglobin 1 alpha chain. The lower band in the left-hand lane represents Hpr alpha chain. The right-hand lane shows that the decidual lysate contains a prominent immunoreactive band in the approximately 30 kDa range.

In liver, haptoglobins are synthesized as a single, contiguous polypeptide chain which is proteolytically cleaved to form the alpha and beta chains. Reaction of the blot with a polyvalent anti-haptoglobin antibody detected a single 45 kDa species. No mature alpha or beta chain was detected.

## Claims

1. A polypeptide having the amino acid sequence: leu tyr ser gly asn asp val thr asp ile ser asp asp arg phe pro lys pro pro glu ile ala asn gly tyr val glu lys leu phe arg tyr gln cys.

2. A substantially pure preparation of a polypeptide, wherein a mammal immunized with said polypeptide produces antibodies which specifically bind an epitope found on a protein comprising the amino acid sequence of claim 1 but not found on haptoglobin 1 or haptoglobin 2, wherein the polypeptide comprises said amino acid sequence.

3. A monoclonal antibody which specifically binds an epitope found on a Hpr protein (cancer related haptoglobin), wherein said Hpr protein is immunologically cross-reactive with the polypeptide of claim 1 but not with haptoglobin 1 or haptoglobin 2.

4. A preparation containing antibodies which specifically bind an epitope found on a Hpr protein comprising the amino acid sequence according to claim 1 but do not specifically bind haptoglobin 1 or haptoglobin 2.

5. The preparation of antibodies according to claim 4, wherein the antibodies are monoclonal antibodies.

6. A method of producing a preparation of a first protein immunologically cross-reactive with the polypeptide of claim 1, comprising:
(a) testing a human breast cancer cell line for the ability to bind to a preparation of antibodies which specifically binds epitopes found on a Hpr protein immunologically cross-reactive with the polypeptide of claim 1 but not found on haptoglobin 1 or 2;
(b) culturing a human breast carcinoma cell line with the ability to bind to said preparation of antibodies;
(c) harvesting said first protein from a cytoplasmic fraction of said cell line, said fraction containing said first protein.

7. A kit for determining the prognosis of breast carcinoma, comprising:
(a) a preparation of antibodies which is immunoreactive with epitopes found on a Hpr protein immunologically cross-reactive with the polypeptide of claim 1 but not found on haptoglobin 1 or 2;
(b) means for detecting the antibodies of said preparation.

8. The kit of claim 7, comprising:
(a) monoclonal antibodies which specifically bind an epitope found on a Hpr protein, wherein said Hpr protein comprises the amino acid sequence of the polypeptide of claim 1 but not with haptoglobin 1 or haptoglobin 2; and
(b) means for detecting the antibodies of said preparation.

9. The kit of claim 7, comprising:
(a) a preparation of antibodies containing antibodies which specifically bind an epitope found on a Hpr protein comprising the amino acid sequence according to claim 1 but do not specifically bind haptoglobin 1 or haptoglobin 2; and
(b) means for detecting the antibodies of said preparation.

10. The kit of claim 7, wherein the means for detecting comprises:
(a) anti-Ig antibodies immunoreactive with the antibodies of said preparation;
(b) a peroxidase enzyme;
(c) a chromogenic substance which changes color in the presence of peroxidase and hydrogen peroxide.

11. The kit of claim 10, wherein the chromogenic substance is aminoethylcarbazole.

12. The kit of claim 7, wherein the means for detecting comprises a radiolabel.

13. The kit of claim 10, wherein the peroxidase enzyme is coupled to the anti-Ig antibodies which are immunoreactive with the antibodies of said preparation.

14. The kit of claim 10, wherein the peroxidase enzyme is complexed to anti-peroxidase antibodies, said anti-peroxidase antibodies being immunoreactive with the anti-Ig antibodies which are immunoreactive with the antibodies of said preparation.

15. A method of determining the prognosis of a solid tumor, comprising:
(a) contacting a histologic section from a solid tumor with a preparation of antibodies which specifically binds a Hpr protein immunologically cross-reactive with the polypeptide of claim 1 but which do not bind haptoglobin 1 or 2;
(b) determining whether the antibodies of said preparation bind to the histologic section, the binding of said antibodies to the histological section correlating with a worsened prognosis of the solid tumor.

16. The method of determining the prognosis of a solid tumor according to claim 15, comprising:
(a) contacting a histologic section from a solid tumor with monoclonal antibodies which specifically binds an epitope found on a Hpr protein, wherein said Hpr protein is immunologically cross-reactive with the polypeptide of claim 1 but not with haptoglobin 1 or haptoglobin 2; and
(b) determining whether the antibodies of said preparation bind to the histologic section, the binding of said antibodies to the histological section correlating with a worsened prognosis of the solid tumor.

17. The method of claim 15, wherein the solid tumor is a breast carcinoma.

18. A method of assaying a first protein immunologically cross-reactive with the polypeptide of claim 1 in a biological fluid, comprising:
(a) providing a solid support with antibodies immunoreactive with an epitope found on a Hpr protein but not found on haptoglobin 1 or haptoglobin 2, said antibodies attached to said solid support;
(b) contacting said solid support with a biological fluid containing an unknown quantity of said first protein under conditions where stable antibody-antigen complexes form between said antibodies and said first protein, and where said antibodies do not react with haptoglobin 1 or haptoglobin 2, to form antibody-protein complexes;
(c) further contacting said solid support obtained in step (b) with a polypeptide containing an epitope found on said Hpr protein but not found on haptoglobin 1 or haptoglobin 2, under said conditions to form antibody-polypeptide complexes, said polypeptide bearing a detectable moiety;
(d) detecting and quantitating said polypeptide having a detectable moiety which in step (c) is bound by means of antibody-antigen complexes to the solid support;
(e) comparing the quantity (1) of polypeptide bound to the solid support in steps (a) through (d) to the quantity (2) of polypeptide which is bound when step (b) is omitted; the reduction in binding of quantity (1) relative to quantity (2) being correlated to the amount of said protein in the biological fluid.

19. The method of claim 18, wherein said antibodies are specifically immunoreactive with said Hpr protein but not haptoglobin 1 or haptoglobin 2.

20. A method of detecting proliferation of tumor cells in a patient, comprising:
(a) quantitating, in a biological fluid from a patient bearing a solid tumor, the amount of a first protein which is immunologically bound by antibodies that specifically bind a Hpr protein immunologically cross-reactive with the polypeptide of claim 1 but do not specifically bind haptoglobin 1 or haptoglobin 2;
(b) comparing the amount of said first protein in the fluid from said patient to the amount found in control fluid collected from a patient with no detectable solid tumor, an increased amount of said first protein indicating proliferation of the tumor cells.

21. The method of claim 20, wherein said quantitating step further comprises binding said first protein by a preparation of antibodies specifically reactive with said Hpr protein.

22. The method of claim 20, wherein the control fluid is from said patient bearing a solid tumor collected at an earlier time.

23. The method of claim 20, wherein the biological fluid is blood plasma or serum.

24. The method of claim 20, wherein the solid tumor is breast carcinoma.

25. A method of detecting proliferation of tumor cells in a patient, comprising:
(a) quantitating, in a sample obtained from the tumor of a patient bearing a solid tumor, the amount of a first protein which is immunologically bound by antibodies that specifically bind a Hpr protein immunologically cross-reactive with the polypeptide of claim 1 but do not specifically bind haptoglobin 1 or haptoglobin 2;
(b) comparing the amount of said first protein in said sample from said patient to the amount found in a control sample obtained from a patient with no detectable solid tumor, an increased amount of said first protein indicating proliferation of the tumor cells.

26. The method of claim 25, wherein the control cell lysate is from said patient bearing a solid tumor collected at an earlier time.

27. The method of claim 25, wherein the solid tumor is breast carcinoma.

28. A method of determining the prognosis of a solid tumor, comprising
(a) quantitating, in a sample obtained from a patient bearing a solid tumor, the amount of a first protein which is immunologically bound by antibodies that specifically bind a Hpr protein immunologically cross-reactive with the polypeptide of claim 1 but do not specifically bind haptoglobin 1 or haptoglobin 2, said sample being a biological fluid of the patient or a solid tissue sample of the patient including a portion of the tumor;
(b) comparing the amount of said first protein in said sample from said patient to the amount found in a control sample, an increased amount of said first protein indicating proliferation of the tumor cells, said control sample being a sample of similar tissue from a control patient with no detectable solid tumor or a portion which does not contain tumor cells from the tissue sample of the patient bearing a solid tumor.

29. The method of claim 28, wherein the solid tumor is breast carcinoma.

## Patentansprüche

1. Polypeptid gekennzeichnet durch die Aminosäuresequenz: leu tyr ser gly asn asp val thr asp ile ser asp asp arg phe pro lys pro pro glu ile ala asn gly tyr val glu lys leu phe arg tyr gln cys.

2. Im wesentlichen reines Präparat eines Polypeptids, dadurch gekennzeichnet, daß ein mit dem besagten Polypeptid immunisiertes Säugetier Antikörper produziert, die ein Epitop in spezifischer Weise binden, das Epitop gründet auf einem aus der Aminosäuresequenz nach Anspruch 1 bestehenden Protein aber nicht auf einem Haptoglobin 1 oder Haptoglobin 2, worin das Polypeptid aus der besagten Aminosäuresequenz besteht.

3. Monokloner Antikörper der ein auf einem Hpr-Protein (karzinomverwandtes Haptoglobin) gründendes Epitop in spezifischer Weise bindet, dadurch gekennzeichnet, daß das besagte Hpr-Protein immunologisch kreuzreaktiv mit dem Polypeptid aus Anspruch 1 ist aber nicht mit Haptoglobin 1 oder Haptoglobin 2.

4. Präparat, daß Antikörper beinhaltet, die ein Epitop, das auf einem aus der Aminosäuresequenz nach Anspruch 1 bestehenden Hpr-Protein gründet, in spezifischer Weise binden aber nicht Haptoglobin 1 oder Haptoglobin 2 in spezifischer Weise binden.

5. Präparat aus Antikörpern nach Anspruch 4, dadurch gekennzeichnet, das die Antikörper monoklone Antikörper sind

6. Verfahren zur Herstellung eines Präparates bestehend aus einem ersten Protein, das immunologisch kreuzreaktiv mit dem Polypeptid aus Anspruch 1 ist, bestehend aus:
a) Testen einer menschlichen Brustkarzinomzellenreihe auf die Fähigkeit, daß sie ein Präparat aus Antikörpern bindet, das in spezifischer Weise Epitope bindet, die Epitope gründen auf einem mit dem Polypeptid nach Anspruch 1 immunologisch kreuzreaktiven Hpr-Protein aber nicht auf Haptoglobin 1 oder Haptoglobin 2;
b) Kultivieren einer menschlichen Brustkarzinomzellenreihe mit der Fähigkeit das besagte aus Antikörpern bestehende Präparat zu binden;
c) Ernten des besagten ersten Proteins von einer cytoplasmischen Fraktion der besagten Zellenreihe, die besagte Fraktion beinhaltet das besagte erste Protein.

7. Diagnose satz um die Prognose für Brustkarzinome zu bestimmen, bestehend aus:
a) einem Präparat aus Antikörpern, das immunreaktiv mit Epitopen ist, die Epitope gründen auf einem mit dem Polypeptid aus Anspruch 1 immunologisch kreuzreaktiven Hpr-Protein aber nicht auf Haptoglobin 1 oder Haptoglobin 2;
b) Mitteln um die Antikörper des besagten Präparates festzustellen.

8. Diagnosesatz nach Anspruch 7, bestehend aus:
a) Monoklonen Antikörpern, die in spezifischer Weise ein auf einem Hpr-Protein gründendes Epitop binden, dadurch gekennzeichnet, daß das besagte Hpr-Protein aus der Aminosäuresequenz des Polypeptids nach Anspruch 1 besteht aber nicht aus Haptoglobin 1 oder Haptoglobin 2; und
b) Mitteln um die Antikörper des besagten Präparates festzustellen.

9. Diagnosesatz nach Anspruch 7 bestehend aus:
a) einem Präparat aus Antikörpern das Antikörper beinhaltet, die in spezifischer Weise ein auf einem Hpr-Protein gründendes Epitop binden, aber nicht in spezifischer Weise Haptoglobin 1 oder 2 binden, das Hpr-Protein besteht aus der Aminosäuresequenz entsprechend Anspruch 1; und
b) Mitteln um die Antikörper des besagten Präparates festzustellen.

10. Diagnosesatz nach Anspruch 7, dadurch gekennzeichnet, daß die Mittel für die Feststellung bestehen aus:
a) einem Anti-Ig Antikörperimmunreaktiv mit Antikörpern des besagten Präparates;
b) einem Peroxidaseenzym;
c) einer chromogenen Substanz, die ihre Farbe in Anwesenheit von Peroxidase und Wasserstoffperoxid ändert.

11. Diagnosesatz nach Anspruch 10, dadurch gekennzeichnet, daß die chromogene Substanz ein Aminoäthylcarbazol ist.

12. Diagnosesatz nach Anspruch 7, dadurch gekennzeichnet, daß die Mittel zum Feststellen aus einer Funkmarke bestehen.

13. Diagnosesatz nach Anspruch 10, dadurch gekennzeichnet, daß das Peroxidaseenzym mit den Anti-Ig-Antikörpern verbunden ist, die immunreaktiv mit den Antikörpern des besagten Präparates sind.

14. Diagnosesatz nach Anspruch 10, dadurch gekennzeichnet, daß das Peroxidaseenzym zusammengesetzt ist zu Anti-Peroxidase-Antikörpern, wobei die besagten Anti-Peroxidase-Antikörper immunreaktiv mit den Anti-Ig-Antikörpern sind, die wiederum immunreaktiv mit den Antikörpern des besagten Präparates sind.

15. Diagnosesatz zur Bestimmung der Prognose für einen festen Tumor, bestehend aus:
a) Kontaktieren einer histologischen Sektion eines festen Tumors mit einem Präparat aus Antikörpern, das in spezifischer Weise ein mit dem Polypeptid nach Anspruch 1 immunologisch kreuzreaktives Hpr-Protein bindet, aber das nicht Haptoglobin 1 oder Haptoglobin 2 bindet;
b) Feststellen ob die Antikörper des besagten Präparates sich mit der histologischen Sektion verbinden, die Verbindung der besagten Antikörper mit der histologischen Sektion korreliert mit einer verschlechternden Prognose für den festen Tumor.

16. Verfahren zur Bestimmung der Prognose für einen festen Tumor nach Anspruch 15, bestehend aus:
a) Kontaktieren einer histologischen Sektion eines festen Tumors mit monoklonen Antikörpern, die in spezifischer Weise ein auf einem Hpr-Protein gründendes Epitop binden, dadurch gekennzeichnet, daß das besagte Hpr-Protein immunologisch kreuzreaktiv mit dem Polypeptid aus Anspruch 1 ist, aber nicht mit Haptoglobin 1 oder Haptoglobin 2; und
b) Bestimmen ob die Antikörper des besagten Präparates sich mit der histologischen Sektion verbinden, die Verbindung der besagten Antikörper mit der histologischen Sektion korreliert mit einer verschlechternden Prognose für den festen Tumor.

17. Verfahren nach Anspruch 15, dadurch gekennzeichnet, daß der feste Tumor eine Brustkarzinom ist.

18. Verfahren zur Prüfung eines ersten mit dem Polypeptid nach Anspruch 1 immunologisch kreuzreaktiven Proteins in einer biologischen Flüssigkeit, bestehend aus:
a) Bereitstellen einer festen Trägersubstanz mit Antikörpern, die immunreaktiv mit einem auf einem Hpr-Protein aber nicht auf Haptoglobin 1 oder Haptoglobin 2 gründenden Epitop sind, die besagten Antikörper werden der besagten festen Trägersubstanz beigefügt;
b) Kontaktieren der besagten festen Trägersubstanz mit einer eine unbekannte Menge des besagten ersten Proteins beinhaltenden, biologischen Flüssigkeit, unter Bedingungen bei denen sich stabile Antikörper-Antigen-Komplexe zwischen besagten Antikörpern und besagten ersten Proteinen bilden, und bei denen die besagten Antikörper nicht mit Haptoglobin 1 oder Haptoglobin 2 reagieren, um einen Antikörper-Protein-Komplex zu bilden;
c) weiterem Kontaktieren der besagten aus Schritt b) erhaltenen, festen Trägersubstanz mit einem Polypeptid, das ein auf dem besagten Hpr-Protein aber nicht auf Haptoglobin 1 oder Haptoglobin 2 gründendes Epitop beinhaltet, unter besagten Bedingungen um einen Antikörper-Polypeptid-Komplex zu bilden, wobei das besagte Polypeptid einen feststellbaren Anteil hervorbringt;
d) Feststellen und Quantifizieren des besagten, durch einen feststellbaren Anteil gekennzeichneten Polypeptids, das in Schritt c) durch die Verwendung von Antikörper-Antigen-Komplexen an die feste Trägersubstanz gebunden ist;
e) Vergleichen der Menge (1) des in den Schritten a) bis d) an die feste Trägersubstanz gebundenen Polypeptids mit der Menge (2) an gebundenen Polypeptiden, die aus dem Auslassen des Schritts b) resultieren; die Verringerung der Bindungen von Menge (1) im Verhältnis zu Menge (2) stehen in Korrelation zu der Menge an besagten Proteinen in der biologischen Flüssigkeit.

19. Verfahren nach Anspruch 18, dadurch gekennzeichnet, daß die besagten Antikörper in spezifischer Weise immunreaktiv mit besagtem Hpr-Protein aber nicht mit Haptoglobin 1 oder Haptoglobin 2 sind.

20. Verfahren zur Bestimmung der Ausbreitung von Tumorzellen in einem Patienten, bestehend aus:
a) Quantifizieren der Menge eines ersten, durch Antikörper immunologisch gebundenen Proteins in einer biologischen Flüssigkeit eines durch einen festen Tumor betroffenen Patienten, die Antikörper binden in spezifischer Weise ein mit dem Polypeptid nach Anspruch 1 immunologisch kreuzreaktives Hpr-Protein, aber binden nicht in spezifischer Weise ein Haptoglobin 1 oder Haptoglobin 2;
b) Vergleichen der Menge des besagten ersten Proteins in der Flüssigkeit des besagten Patienten mit der Menge, die in einer Kontrollflüssigkeit eines Patienten ohne einen feststellbaren festen Tumors gefunden wurde, wobei ein Anstieg der Menge des besagten ersten Proteins eine Ausbreitung der Tumorzellen anzeigt.

21. Verfahren nach Anspruch 20, dadurch gekennzeichnet, daß besagter quantifizierender Schritt weiter beinhaltet das Binden des besagten ersten Proteins durch ein Präparat bestehend aus in spezifischer Weise mit dem besagten Hpr-Protein reaktiven Antikörpern.

22. Verfahren nach Anspruch 20, dadurch gekennzeichnet, daß die Kontrollflüssigkeit zu einem früheren Zeitpunkt vom besagten Patienten stammt, der durch einen festen Tumor betroffen ist.

23. Verfahren nach Anspruch 20, dadurch gekennzeichnet, daß die biologische Flüssigkeit Blutplasma oder Serum ist.

24. Verfahren nach Anspruch 20, dadurch gekennzeichnet, daß der feste Tumor ein Brustkarzinom ist.

25. Verfahren zur Bestimmung der Ausbreitung von Tumorzellen in einem Patienten, bestehend aus:
a) Quantifizieren der Menge eines ersten, durch Antikörper immunologisch gebundenen Proteins in einer Tumorprobe eines durch einen festen Tumor betroffenen Patienten, die Antikörper binden in spezifischer Weise ein mit dem Polypeptid nach Anspruch 1 immunologisch kreuzreaktives Hpr-Protein aber binden nicht in spezifischer Weise ein Haptoglobin 1 oder Haptoglobin 2;
b) Vergleichen der Menge des besagten Proteins in der erste Probe des besagten Patienten mit der Menge, die in einer Kontrollprobe eines Patienten ohne einen feststellbaren festen Tumors gefunden wurde, wobei ein Anstieg der Menge des besagten ersten Proteins eine Ausbreitung der Tumorzellen anzeigt.

26. Verfahren nach Anspruch 25, dadurch gekennzeichnet, daß die Kontrollzellenlymphe zu einem früheren Zeitpunkt vom besagten Patienten stammt, der durch einen festen Tumor betroffen ist.

27. Verfahren nach Anspruch 26, dadurch gekennzeichnet, daß der feste Tumor ein Brustkarzinom ist.

28. Verfahren zur Bestimmung der Prognose eines festen Tumors gekennzeichnet durch:
a) Quantifizieren der Menge eines ersten, durch Antikörper immunologisch gebundenen Proteins in einer Probe eines durch einen festen Tumor betroffenen Patienten, die Antikörper binden in spezifischer Weise ein mit dem Polypeptid nach Anspruch 1 immunologisch kreuzreaktives Hpr-Protein aber binden nicht in spezifischer Weise ein Haptoglobin 1 oder Haptoglobin 2, wobei die besagte Probe eine biologische Flüssigkeit des Patienten oder eine feste, einen Tumoranteil beinhaltende Gewebeprobe des Patienten ist;
b) Vergleichen der Menge des besagten ersten Proteins in der besagten Probe des besagten Patienten mit der Menge, die in der Kontrollprobe gefunden wurde, ein Anstieg der Menge des besagten ersten Proteins eine Ausbreitung der Tumorzellen anzeigt, die besagte Kontrollprobe eine Probe eines ähnlichen Gewebes von einem Kontrollpatienten ist, bei dem kein feststellbarer fester Tumor gefunden wurde, oder es sich um eine tumorzellenfreie Kontrollprobe handelt, die von der Gewebeprobe des durch einen festen Tumor betroffenen Patienten stammt.

29. Verfahren nach Anspruch 28, dadurch gekennzeichnet, daß der feste Tumor ein Brustkarzinom ist.

## Revendications

1. Polypeptide ayant la séquence d'acides aminés suivante : leu tyr ser gly asn asp val thr asp ile ser asp asp arg phe pro lys pro pro glu ile ala asn gly tyr val glu lys leu phe arg tyr gln cys.

2. Préparation pratiquement pure d'un polypeptide, où un mammifère immunisé avec ledit polypeptide produit des anticorps qui se lient spécifiquement à un épitope présent sur une protéine comprenant la séquence d'acides aminés de la revendication 1 mais ne se trouvant pas sur l'haptoglobuline 1 ni l'haptoglobuline 2, où le polypeptide comprend ladite séquence d'acides aminés.

3. Anticorps monoclonal qui se lie spécifiquement à un épitope trouvé sur une protéine Hpr (haptoglobuline apparentée au cancer ou "cancer related haptoglobulin"), où ladite protéine Hpr réagit immunologiquement de façon croisée avec le polypeptide de la revendication 1 mais pas avec l'haptoglobuline 1 ni l'haptoglobuline 2.

4. Préparation contenant des anticorps qui se lient spécifiquement à un épitope présent sur une protéine Hpr comprenant la séquence d'acides aminés selon la revendication 1 mais ne se lient pas spécifiquement à l'haptoglobuline 1 ni l'haptoglobuline 2.

5. Préparation d'anticorps selon la revendication 4, où les anticorps sont des anticorps monoclonaux.

6. Méthode de production d'une préparation d'une première protéine qui réagit immunologiquement de façon croisée avec le polypeptide de la revendication 1, comprenant :
(a) l'analyse de la capacité d'une lignée cellulaire humaine de cancer du sein à se lier à une préparation d'anticorps qui se lient spécifiquement aux épitopes présents sur une protéine Hpr qui réagit immunologiquement de façon croisée avec le polypeptide de la revendication 1, mais qui ne se trouvent pas sur l'haptoglobuline 1 ni l'haptoglobuline 2 ;
(b) la culture d'une lignée cellulaire de carcinome du sein humaine ayant la capacité de se lier à ladite préparation d'anticorps ;
(c) la récupération de ladite première protéine d'une fraction cytoplasmique de ladite lignée cellulaire, ladite fraction contenant ladite première protéine.

7. Trousse pour réaliser le pronostic du carcinome du sein, comprenant :
(a) une préparation d'anticorps qui est immunoréactive avec des épitopes présents sur une protéine Hpr qui réagit immunologiquement de façon croisée avec le polypeptide de la revendication 1, mais qui ne se trouvent pas sur l'haptoglobuline 1 ni l'haptoglobuline 2 ;
(b) des moyens pour détecter les anticorps de ladite préparation.

8. Trousse selon la revendication 7, comprenant :
(a) des anticorps monoclonaux qui se lient spécifiquement à un épitope présent sur une protéine Hpr, où ladite protéine Hpr comprend la séquence d'acides aminés du polypeptide de la revendication 1 mais ne se lient pas à l'haptoglobuline 1 ni l'haptoglobuline 2 ; et
(b) des moyens pour détecter les anticorps de ladite préparation.

9. Trousse selon la revendication 7, comprenant :
(a) une préparation d'anticorps contenant des anticorps qui se lient spécifiquement à un épitope présent sur une protéine Hpr comprenant la séquence d'acides aminés selon la revendication 1 mais ne se lient pas à l'haptoglobuline 1 ni l'haptoglobuline 2 ; et
(b) des moyens pour détecter les anticorps de ladite préparation.

10. Trousse selon la revendication 7, où les moyens pour la détection comprennent :
(a) des anticorps anti-Ig immunoréactifs envers les anticorps de ladite préparation ;
(b) une enzyme péroxidase ;
(c) une substance chromogénique qui change de couleur en présence de péroxidase et de péroxide d'hydrogène.

11. Trousse selon la revendication 10, où la substance chromogénique est l'aminoéthylcarbazole.

12. Trousse selon la revendication 7, où les moyens pour la détection comprennent un marqueur isotopique.

13. Trousse selon la revendication 10, où l'enzyme péroxidase est couplée aux anticorps anti-Ig qui sont immunoréactifs envers les anticorps de ladite préparation.

14. Trousse selon la revendication 10, où l'enzyme peroxydase est complexée à des anticorps anti-peroxidase, lesdits anticorps anti-peroxidase étant immunoréactifs envers les anticorps anti-Ig qui sont immunoréactifs envers les anticorps de ladite préparation.

15. Méthode pour réaliser le pronostic d'une tumeur solide comprenant :
(a) la mise en contact d'une coupe histologique d'une tumeur solide avec une préparation d'anticorps qui se lient spécifiquement à une protéine Hpr qui réagit immunologiquement de façon croisée avec le polypeptide de la revendication 1 mais qui ne se lient pas à l'haptoglobuline 1 ni 2 ;
(b) la détermination de la liaison des anticorps de ladite préparation à la coupe histologique, la liaison desdits anticorps à la coupe histologique étant corrélée avec un diagnostic de la tumeur solide aggravé.

16. Méthode pour réaliser le diagnostic d'une tumeur solide selon la revendication 15, comprenant :
(a) la mise en contact d'une coupe histologique d'une tumeur solide avec des anticorps monoclonaux qui se lient spécifiquement à un épitope présent sur une protéine Hpr, où ladite protéine Hpr réagit immunologiquement de façon croisée avec le polypeptide de la revendication 1 mais pas avec l'haptoglobuline 1 ni l'haptoglobuline 2 ; et
(b) la détermination de la liaison des anticorps de ladite préparation à la coupe histologique, la liaison desdits anticorps à la coupe histologique étant corrélée avec un pronostic de tumeur solide aggravé.

17. Méthode selon la revendication 15, où la tumeur solide est un carcinome du sein.

18. Méthode de dosage d'une première protéine qui réagit immunologiquement de façon croisée avec le polypeptide de la revendication 1 dans un fluide biologique, comprenant :
(a) la fourniture d'un support solide avec des anticorps immunoréactifs envers un épitope présent sur une protéine Hpr mais ne se trouvant pas sur l'haptoglobuline 1 ni l'haptoglobuline 2, lesdits anticorps étant fixés audit support solide ;
(b) la mise en contact dudit support solide avec un fluide biologique contenant une quantité inconnue de ladite première protéine dans des conditions où des complexes stables anticorps-antigène se forment entre lesdits anticorps et ladite première protéine, et où lesdits anticorps ne réagissent pas avec l'haptoglobuline 1 ni l'haptoglobuline 2, pour former des complexes anticorps-protéines ;
(c) la mise en contact ultérieure dudit support solide obtenu dans l'étape (b) avec un polypeptide contenant un épitope présent sur ladite protéine Hpr mais qui ne se trouve pas sur l'haptoglobuline 1 ni l'haptoglobuline 2, dans lesdites conditions pour former des complexes anticorps-polypeptides, ledit polypeptide portant une portion détectable ;
(d) la détection et la quantification dudit polypeptide ayant une portion détectable qui dans l'étape (c) est liée au support solide par des complexes anticorps-antigènes ;
(e) la comparaison de la quantité (1) de polypeptide lié au support solide dans les étapes (a) à (d) avec la quantité (2) de polypeptide qui est lié lorsque l'étape (b) n'est pas réalisée ; la réduction dans la liaison de la quantité (1) par rapport à la quantité (2) étant corrélée à la quantité de ladite protéine dans le fluide biologique.

19. Méthode selon la revendication 18, où lesdits anticorps sont spécifiquement immunoréactifs envers ladite protéine Hpr mais pas envers l'haptoglobuline 1 ni l'haptoglobuline 2.

20. Méthode pour la détection de la prolifération de cellules tumorales chez un patient, comprenant :
(a) la quantification, dans un fluide biologique provenant d'un patient ayant une tumeur solide, de la quantité d'une première protéine qui est immunologiquement fixée par des anticorps qui se lient spécifiquement à une protéine Hpr qui réagit immunologiquement de façon croisée avec le polypeptide de la revendication 1, mais ne se lient pas spécifiquement à l'haptoglobuline 1 ni l'haptoglobuline 2 ;
(b) la comparaison de la quantité de ladite première protéine dans le fluide dudit patient à celle trouvée dans un fluide témoin prélevé chez un patient sans tumeur solide détectable, une augmentation de la quantité de ladite première protéine indiquant la prolifération des cellules tumorales.

21. Méthode selon la revendication 20, où ladite étape de quantification comprend en outre la fixation de ladite première protéine par une préparation d'anticorps spécifiquement réactifs envers ladite protéine Hpr.

22. Méthode selon la revendication 20, où le fluide témoin provient dudit patient présentant une tumeur solide collecté à une période plus précoce.

23. Méthode selon la revendication 20, où le fluide biologique est du plasma sanguin ou du sérum.

24. Méthode selon la revendication 20, où la tumeur solide est un carcinome du sein.

25. Méthode de détection de la prolifération de cellules tumorales chez un patient, comprenant :
(a) la quantification, dans un échantillon obtenu à partir de la tumeur d'un patient ayant une tumeur solide, de la quantité d'une première protéine qui est immunologiquement fixée par des anticorps qui se lient spécifiquement à une protéine Hpr qui réagit immunologiquement de façon croisée avec le polypeptide de la revendication 1 mais ne se lient pas spécifiquement à l'haptoglobuline 1 ni l'haptoglobuline 2 ;
(b) la comparaison de la quantité de ladite première protéine dans ledit échantillon provenant dudit patient à celle trouvée dans un échantillon témoin obtenu chez un patient qui n'a pas de tumeur solide détectable, une augmentation de la quantité de ladite première protéine étant une indication de la prolifération des cellules tumorales.

26. Méthode selon la revendication 25, où le lysat cellulaire témoin provient dudit patient présentant une tumeur solide, collecté à une période plus précoce.

27. Méthode selon la revendication 25, où la tumeur solide est un carcinome du sein.

28. Méthode pour réaliser un pronostic d'une tumeur solide, comprenant
(a) la quantification, dans un échantillon obtenu à partir d'un patient ayant une tumeur solide, de la quantité d'une première protéine qui est immunologiquement fixée par des anticorps qui se lient spécifiquement à une protéine Hpr qui réagit immunologiquement de façon croisée avec le polypeptide de la revendication 1 mais ne se lient pas spécifiquement à l'haptoglobuline 1 ni l'haptoglobuline 2, ledit échantillon étant un fluide biologique du patient ou un échantillon de tissu solide du patient, incluant un fragment de la tumeur ;
(b) la comparaison de la quantité de ladite première protéine dans ledit échantillon provenant de ce patient par rapport à la quantité trouvée dans un échantillon témoin, une augmentation de la quantité de ladite première protéine étant une indication de la prolifération des cellules tumorales, ledit échantillon témoin étant un échantillon d'un tissu similaire provenant d'un patient témoin sans tumeur solide détectable ou une portion qui ne contient pas de cellule tumorale provenant d'un échantillon de tissu du patient ayant une tumeur solide.

29. Méthode selon la revendication 28, où la tumeur solide est un carcinome du sein.
